# EUROPEAN PATENT APPLICATION

(11) **EP 0 868 892 A1**
(43) Date of publication of application: **07.10.1998**
(21) Application number: 98302403.5
(22) Date of filing: 30.03.1998
(51) Int. Cl.: A61F 5/441

(54) **Venting valve for ostomy bag**

(30) Priority: 02.04.1997 GB 9706655
(71) Applicant: BRISTOL-MYERS SQUIBB COMPANY, Skillman, NJ 08558 (US)
(72) Inventor: Falconer, Malcolm Ian, Wandsworth Common, London SW17 7DG (GB)
(74) Representative: Holmes, Miles Keeton

(57) **Abstract**

A valve for an ostomy bag includes a housing (10) attached or attachable to the bag and a valve member (24), the valve member being movable out of predetermined sealing engagement with an interior surface of the housing to open a gas escape path through apertures (18) upon depression of a deformable button portion (12) of the housing. The valve member may comprise a hinged diaphragm, or a centrally mounted plate. The housing may be fitted over a filter element (36), or the filter element may be contained within the housing. The housing could be part of a bagside coupling member, or may include an arrangement for puncturing a hole in the bag when the valve is fitted thereto.

## Description

This invention relates to a valve for an ostomy bag. Such valves are used to enable a user to regulate manually the venting of flatus from the bag.

Current ostomy pouches have a filter which vents gases from the bag, stopping the bag from inflating. However, constant venting can have a reverse effect by causing the bag to collapse by the bag sides sticking together. This would hinder evacuation of the stomal discharge into the bag. Therefore, provision of a valve can ensure development of sufficient gas pressure to prevent sticking together of the bag walls, while providing a user controllable vent to prevent over inflation.

EP-A-0 653 196 describes a known ostomy vent valve which includes a small rotatable handle, and a valve member which is pressed resiliently over an aperture on the bag side part of the valve. Such an arrangement may require considerable dexterity to be controlled accurately by elderly or infirm users. Furthermore, the fact that the valve member is urged resiliently in a counter direction to the gas pressure means that considerable resilient force must be required to achieve a reliable gas tight seal. Such a high closure force is likely to hinder movement of the rotatable handle, and make it even more difficult to be controlled by elderly or infirm users.

Reference is also made to US-A-4,232,672, US-A4,451,258, US 4,938,749,US 4,810,250, US 4,863,447, EP-A-0 535 801, GB-A-2300359 and GB-A-2280611 which illustrate other venting arrangements.

In contrast, the invention provides a valve for an ostomy bag, the valve comprising a housing attached or attachable to an ostomy bag and a valve member, the valve member being movable out of predetermined sealing engagement with the housing to open a gas escape path upon depression of a deformable portion (for example, a button portion) of the housing.

Such an arrangement can provide a valve which is simple to construct, and is very straightforward to use. Furthermore, in use, the valve member does not need to act against the gas pressure to form a seal; the gas pressure can act on the valve in a direction which improves the seal with increasing pressure. Therefore, the user does not have to overcome any strong resilient forces in the valve member itself before the valve can be opened.

The valve may be attached directly or indirectly to the pouch. In some of the preferred embodiments, the valve is mounted on a base which is mounted on the pouch.

In a further aspect, the invention provides a valve for an ostomy bag, the valve comprising a housing attached or attachable to an ostomy bag, and a valve member, the housing comprising a cover member positioned over the valve member, and at least a portion of the valve member being movable out of predetermined sealing engagement with an interior surface of the cover member to open a gas escape path upon depression of a deformable portion of the cover member.

Embodiments of the invention are now described by way of example only, with reference to the accompanying drawings, in which:
Figure 1 is a schematic sectional view of a first embodiment of an ostomy bag valve, in its normal closed state;
Figure 2 is a schematic sectional view of the valve in an open state;
Figure 3 is a plan view of the valve cover in isolation; and
Figure 4 is a plan view of the valve flap in isolation;
Figure 5 is a schematic sectional view of a second embodiment of valve;
Figure 6 is a schematic exploded view of the valve of Figure 5;
Figure 7 is an expanded view showing a detail of Figure 6;
Figure 8 is a schematic view of a third embodiment;
Figures 9-12 are schematic views illustrating attachment of a fourth embodiment;
Figures 13 and 14 are schematic views illustrating a further design of venting valve;
Fig. 15 is a cut-off perspective view showing a valve mounted on an ostomy coupling flange; and
Fig. 16 is a schematic section along the line X-X of Fig. 15.

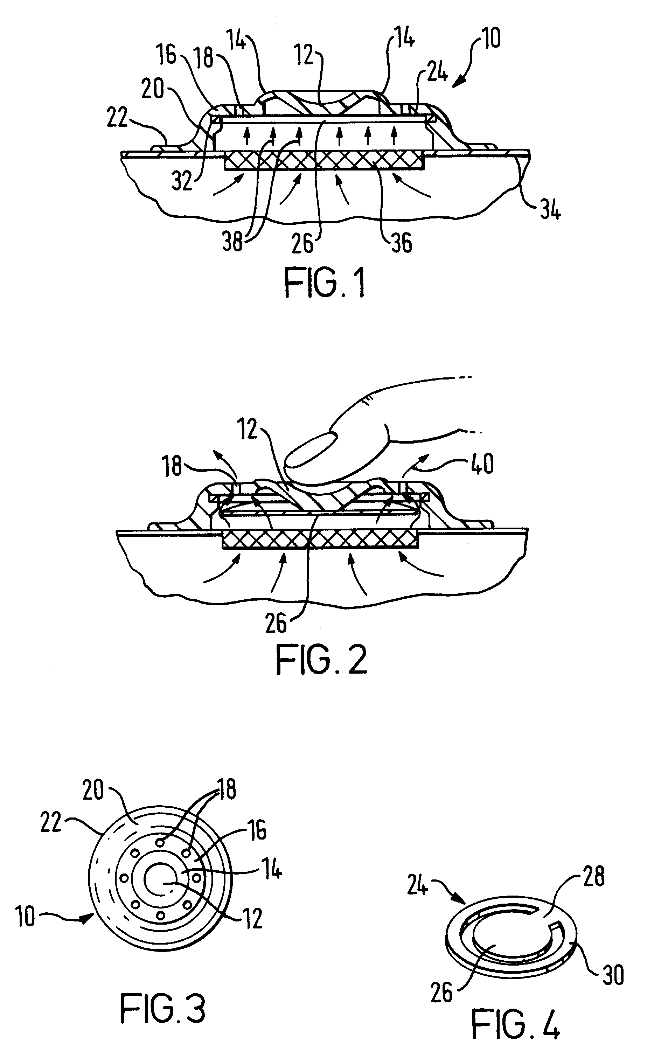

Referring to Figures 1-4, a first embodiment of valve assembly comprises a housing consisting of a single piece circular cover member 10 of soft plastics material, for example, a flexible thermoplastic elastomer (TPE) material. The cover member 10 has a projecting button 12 with a central depression and is connected to the surrounding part of the cover member by a thin wall portion 14 which permits the button to be depressed by finger pressure. The surrounding part of the cover member includes a generally flat annular wall 16 with a plurality of venting apertures 18 spaced regularly on a pitch circle diameter around the button, a peripheral rim 20 and a radially outwardly extending flange 22.

A sealing member 24 (herein also called a valve member) is fitted inside the cover member 10 for operation by the button 12. The sealing member consists of a central disc shaped flap 26 which is integrally joined by a narrow hinge web 28 to a surrounding annular mounting ring 30. In this embodiment, the sealing member 24 is punched from a single sheet of material, which may, for example, be of mica or of polycarbonate.

The ring 30 of the sealing member 24 is received in an undercut 32 on the interior surface of the cover member 10 immediately behind the annular wall 16. The flap 26 is dimensioned to be larger than the pitch circle of the apertures 18, such that, in a normal state, the flap 26 lies against the underside of annular wall 16 and blocks the apertures 18.

The valve assembly may be attached in any suitable manner over a conventional ostomy filter 36 carried on, or in, an ostomy bag wall 34. For example, the cover member may be attached by welding around the flange 22, or by the use of suitable adhesive tape between the flange 22 and the ostomy bag wall 34. The filter 36 may also be attached to the bag wall 34 in any suitable manner, for example, by welding around its periphery.

Referring to Fig. 1, in use at very low gas pressure inside the ostomy bag, the flap 26 lying against the underside of the annular wall 16 and blocking the apertures 18 provides a seal to prevent the venting of gas through the valve. As more gas is discharged into the bag, and the gas pressure rises, the pressure acts on the flap in the direction indicated by arrows 38, thereby holding the flap even more firmly against the annular wall 16, and increasing the effectiveness of the seal against leaks.

Referring to Fig. 2, to open the valve, the user presses the button 12 inwardly, causing it to bear against the flap 26, and deflect the flap 26 downwardly away from the annular wall 16. This unblocks the apertures 18, and allows gas to vent around the periphery of the flap and through the apertures, as indicated by arrows 40.

When the button 12 is released by the user, the button and flap 26 return to their natural positions shown in Fig. 1 by their resilience. If any positive gas pressure remains, then this also assists closing of the valve by urging the flap 24 against the underside of the annular wall 16 when the button 12 is released.

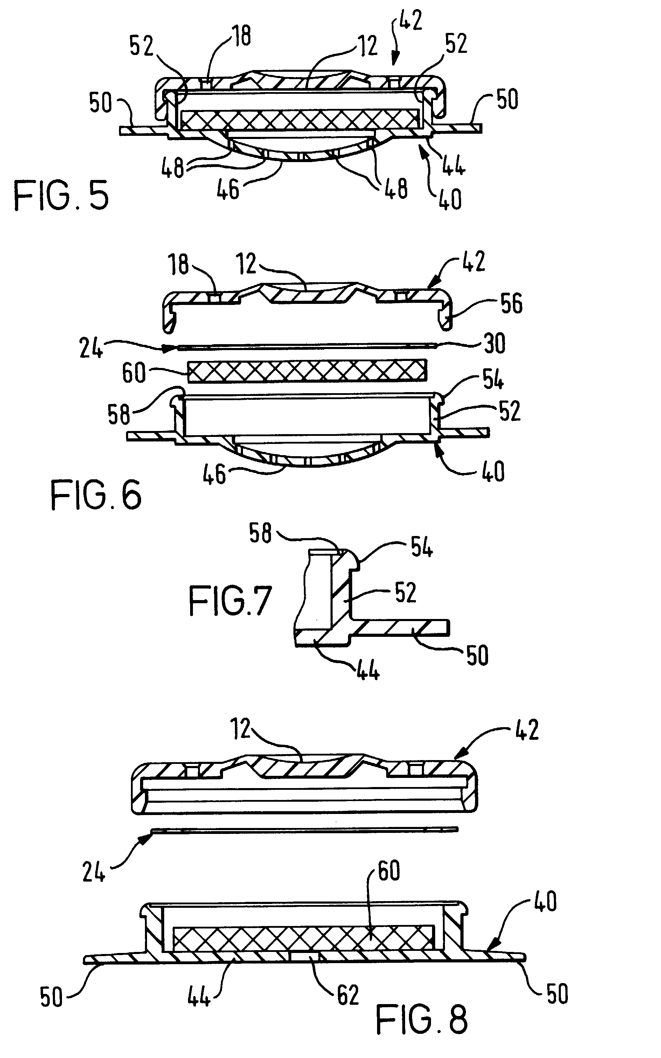

Figures 5-7 illustrate a modified second embodiment of valve. The valve is similar to that described above, and the same reference numerals denote corresponding features, where appropriate.

The principal difference in the second embodiment resides in the structure of the valve housing. The housing consists of a rear part 40 to which a front cover 42 is attached in a clip-on manner. The rear part 40 comprises a rear wall 44 with a rearward bulge 46 having perforations 48 to allow gas to enter the housing. The rear part 40 also has a radial flange 50 to allow the housing to be attached to an ostomy bag, for example by welding or by adhesive tape, as with the previous embodiment. A cylindrical wall 52 projects from the rear part 40 for mounting the front cover 42. The cylindrical wall 52 has a radial lip 54 which engages behind a corresponding lip 56 of the front cover 42 with a snap fit when the cover 42 is pushed onto the rear part 40. The peripheral mounting ring 30 of the sealing member 24 is received within an annular recess 58 of the cylindrical wall, and is trapped in position by the front cover 42 when assembled. The sealing member performs in the same manner as that described in the first embodiment, and is opened by the application of finger pressure to the button 12, to allow gas to escape through venting apertures 18.

Since in this embodiment, the housing has a rear wall 44, the housing can contain a filter element 60 adhered to the rear wall 44. The assembled valve and filter can be secured to the wall material for the ostomy bag as a ready-assembled item, or the rear housing part 42 can be fitted first, and the filter and valve assembled thereafter.

Figure 8 illustrates a modified third embodiment which is very similar to the embodiment illustrated in Figures 5-7 except that the rear wall 44 of the rear housing part 40 is generally flat. The rear wall 44 has a central opening 62 to allow gas to enter the valve housing. Such a flat rear wall may simplify welding of the housing to the wall material for an ostomy bag.

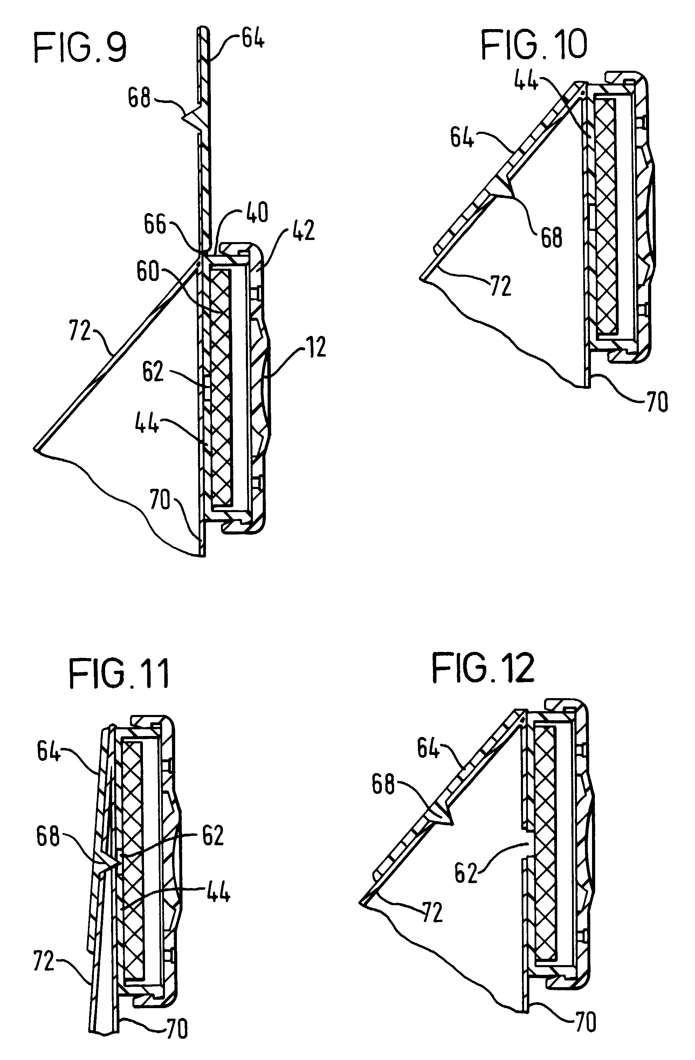

Figures 9-12 illustrate the attachment of a fourth embodiment of valve assembly to an ostomy or other bag. The valve assembly is similar to the third embodiment illustrated in Figure 8, but the peripheral flange 50 is omitted, and the rear housing part 40 has a lateral extension flap 64 which is integrally joined to the rear wall 44 by a flexible hinge web 66. The extension flap 64 carries a sharp projection 68 which, when the flap is folded over towards the rear wall 44, enters the opening 62.

The extension flap may be circular to match the shape of the housing or it may have a different shape. The rear wall 44 and the extension flap 64 carry adhesive to facilitate attachment to an ostomy bag. In use, the housing is fitted to the bag by positioning the housing adjacent to an edge (usually the top edge) of the front wall 70 of the ostomy bag, and then pressing the housing against the front wall 70 so that the rear wall 44 of the housing adheres to the front wall 70 (Fig. 9).

Thereafter, the extension flap 64 is folded over the edge of the ostomy bag to contact the rear wall 72 of the bag (Fig. 10) and to pierce the rear wall 72 with the sharp projection 68.

Referring to Figure 11, the extension flap 64 is further pressed until the sharp projection 68 pierces the front wall 70 of the ostomy bag to create an inlet path for gas into the housing. The bag walls are squeezed between the rear wall 44 of the housing and the extension flap 64, which consolidates the adhesion of the adhesive.

Referring to Figure 12, when the extension flap 64 is released, it relaxes back with the rear wall 72 of the pouch to the normal pouch shape, leaving clear the openings defining the inlet to the housing. The adhesive surrounding the sharp projection 68, and surrounding the opening 62 can ensure a reliable seal to prevent leakage of gas.

It will be appreciated that the fourth embodiment provides a simple and quick technique for attaching the housing to an ostomy bag, and for automatically creating a vent opening in the wall of the bag at the correct position for the housing.

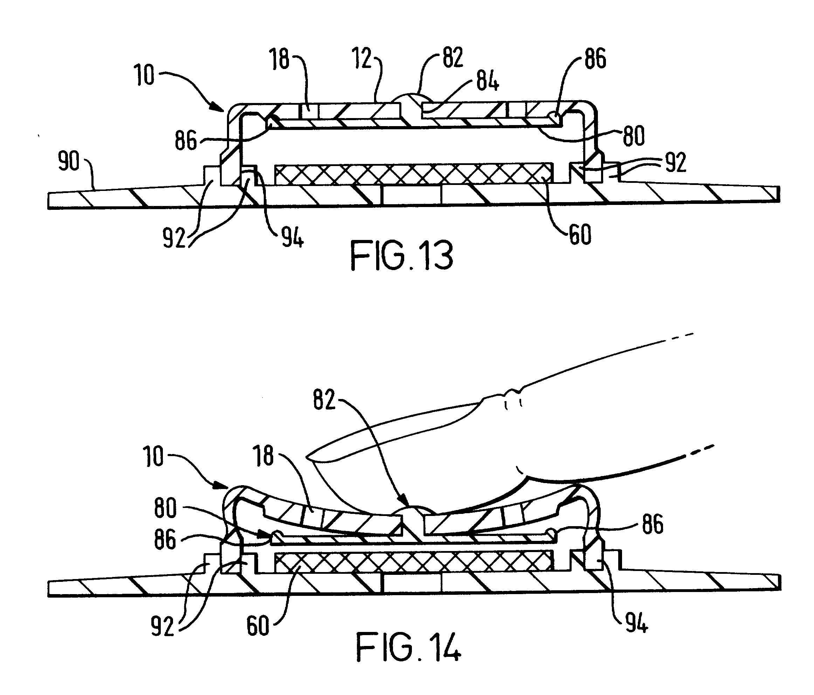

Referring to Figs. 13 and 14, a fifth embodiment of valve is illustrated. This is similar to the valves described previously, and the same reference numerals will be used where appropriate. The principal difference between the fifth embodiment and the earlier embodiments is that the hinged flap is replaced by a non-hinged, generally rigid plate in the form, in this embodiment, of disc 80. The disc may, for example, be made of plastics such as high density polyethylene (HDPE), or of acrylonitrile butadiene styrene (ABS).

Although the cover 10 could have a similar shape to that illustrated in Figs. 1-12, in the present embodiment the cover 10 has a flat-top shape without a button depression region. Instead, the region to press is indicated by the top of a tactile stud 82, described below.

The disc is attached to the central region 12' of the cover 10 to move downwardly therewith when the central region 12' is depressed. In the present embodiment, the disc 80 has an integrally moulded stud 82 which projects upwardly through, and engages in, an opening 84 in the central region 12' to secure the disc 80 to the button.

In normal use, the resilience of the cover member 10 maintains the cover 10 in its upper position (see Fig. 13), such that the disc 80 bears against the interior surface of the cover member 10 to cover the venting apertures 18. As described previously, any positive gas pressure acting on the disc 18 serves to increase the sealing pressure of the disc 80 against the cover member 10.

In order to open the valve, the user presses the central region 12', which causes the disc 80 to move downwardly away from the cover member 10 (see Fig. 14), and thus opens gas escape paths through the venting apertures 18. When the central region 12' is released, the cover 10 returns to its normal position (Fig. 13), thereby moving the disc 80 back into sealing engagement with the interior surface of the cover member 10.

In this embodiment, the disc has an small upstanding peripheral rim 86 which tends to dig in slightly to the surface of the soft material of the cover member 10 when in the closed position. This can provide a pressure point at which the sealing pressure is concentrated to further improve the reliability of the seal.

In this embodiment, the base 90 carries a filter 60 and has twin surrounding walls 92 defining a channel for receiving the edge wall 94 of the cover member 10. The edge 94 of the cover member may be retained in the channel by being a tight friction fit, or by the use of adhesive, or by interlocking (e.g. snap fit) profiles on the edge wall 94 and one or both of the cylindrical walls 92. It will be appreciated that the other methods of attachment described hereinbefore can also be used as desired. It will also be appreciated that the base 90 may have a larger aperture, or may be omitted altogether, to enable the valve unit to be fitted over an existing filter, as in the first embodiment.

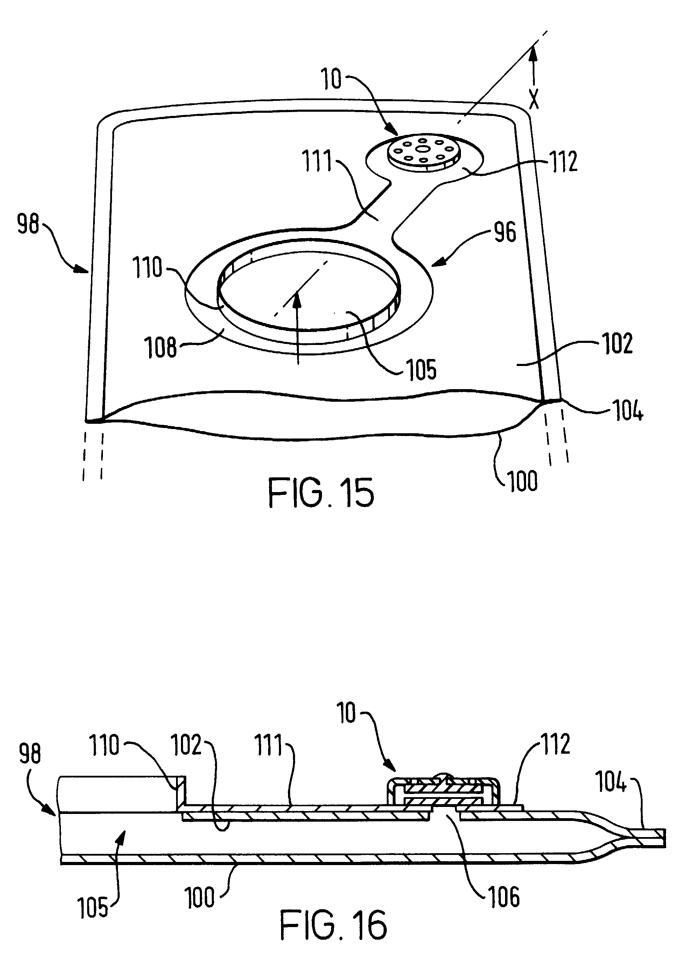

The above embodiments illustrate a dedicated valve unit which is attached to the pouch separately from any coupling arrangement for securing the pouch to the peristomal region of the wearer. However, referring to Figs. 15 and 16, a further embodiment is illustrated in which the valve unit is mounted on a bagside coupling member 96 secured to a pouch 98 by, for example, welding or by adhesive.

The pouch consists generally of a front sheet 100 and a rear sheet 102 both of plastics, and welded together around their periphery 104. The rear sheet 102 includes a conventional stomal aperture 105 for receiving a stomal discharge, and a vent aperture 106 which is normally (but need not necessarily be) above the stomal aperture 105 when the pouch is in its normal upright orientation. The coupling member 96 includes generally circular apertured base 108 carrying a coupling profile 110 for engaging a complementary bodyside coupling member (not shown). Many designs of bagside and bodyside members are known to the skilled man, and so need not be described further. Extending from the base 108 is a generally flat arm 111 including an apertured region 112 for mounting the cover 10 of the valve unit in register with the vent aperture 106. The cover member 10 is secured to the mounting region 112, for example, by welding or by adhesive, or by an interference fit. The cover member 10 may house a hinged diaphragm valve (as in the first four embodiments) or as illustrated houses a non-hinged plate (as in the fifth embodiment). The arm 111 is generally non-rigid so as not to impede flexing of the bag in use. However, the arm may provide some support for the filter unit to prevent any tendency of the bag to become squashed when a user applies finger pressure to open the valve; it will be appreciated that the additional contact area between the coupling member 96 and the rear wall 102 of the pouch 98 can spread the reaction force over a relatively larger area of the pouch wall 102, thus reducing the tendency for localised collapsing together of the pouch walls in the region of the filter unit. Also, at least some of the reaction force could be borne through the bagside and bodyside coupling members, if desired.

Although in the illustrated embodiment, the filter unit is mounted on an arm 111 and is spaced from the main base 108 of the coupling member 96, it will be appreciated that in other designs, the filter unit could be mounted on the base 108, for example, on an enlarged peripheral region of the base. This could provide additional mechanical support for the filter unit, as described above.

It will be appreciated that the invention, particularly as illustrated in the preferred embodiments, enables a simple but very effective ostomy valve to be constructed, which can be cheap and easy to produce, and very straightforward to operate. The user has simply to press the central button 12 to open the valve, which is self closing upon release of the button. Furthermore, since the valve uses positive gas pressure to increase the sealing pressure of the valve, the valve does not need a strong spring action, and it therefore needs only modest finger pressure to operate.

## Claims

1. A valve for an ostomy bag, the valve comprising a housing attached or attachable to an ostomy bag and a valve member, the valve member being movable out of predetermined sealing engagement with the housing to open a gas escape path upon depression of a deformable portion of the housing.

2. A valve according to claim 1, wherein the housing comprises a cover member positioned over the valve member, and at least a portion of the valve member being movable out of predetermined sealing engagement with an interior surface of the cover member to open a gas escape path upon depression of a deformable portion of the cover member.

3. A valve according to claim 2, wherein the cover member comprises one or more venting apertures through which gas can escape when the valve member is moved out of said predetermined sealing engagement.

4. A valve according to claim 3, comprising a plurality of said apertures arranged in the cover member in or around the deformable portion.

5. A valve according to claim 4, wherein the valve member underlies the apertures to close the apertures when in sealing engagement with the interior surface of the cover member.

6. A valve according to any preceding claim, wherein the valve member is attached to and supported by the housing.

7. A valve member according to any preceding claim, wherein the valve member comprises a mounting portion and a diaphragm flap integrally hinged to the mounting portion, the diaphragm flap being hingedly movable out of said predetermined sealing engagement with the housing to open the gas escape path.

8. A valve according to claim 7, wherein the mounting portion extends around the periphery of the flap.

9. A valve according to claim 8, wherein the flap is generally circular, and the mounting portion comprises a ring surrounding the flap, the ring and the flap being integrally joined in a first region to allow deflection of the flap from the plane of the ring.

10. A valve according to claim 7, 8 or 9, wherein the mounting portion of the valve member is received in a recess of the housing.

11. A valve according to any of claims 7 to 10, wherein, in use, the resilience of the integral hinge returns the flap to the predetermined sealing position when the external pressure applied to the button portion of the cover member is released.

12. A valve according to claim 6, wherein the valve member comprises a generally rigid plate supported by the cover member.

13. A valve according to claim 12, wherein the plate is centrally mounted.

14. A valve according to claim 12 or 13, wherein the plate comprises a mounting projection received in an opening or recess of the cover member.

15. A valve according to claim 14, wherein the projection comprises a stud having an undercut.

16. A valve according to any preceding claim, wherein the valve member is punched from sheet material.

17. A valve as defined in any preceding claim, wherein the deformable portion of the housing comprises a finger operated button portion.

18. A valve as defined in any preceding claim, wherein the front cover member is attachable or attached to a rear housing part.

19. A valve according to claim 18, wherein the rear housing part comprises a radial flange for welding to an ostomy pouch.

20. A valve as defined in any of claims 1 to 18 wherein the housing comprises a hinged wall carrying a sharp projection such that, in use, the housing can be fitted adjacent to an edge of an ostomy bag and the hinged wall folded over the edge to punch a hole through the walls of the ostomy bag to allow gas to enter the housing.

21. A valve according to claim 20, wherein the hinged wall and a rear face of the housing carry adhesive.

22. A valve according to any preceding claim, wherein the housing contains or receives a filter.

23. A bagside coupling member for an ostomy pouch, the bagside coupling member carrying a vent valve as defined in any preceding claim.

24. An ostomy pouch comprising a bagside coupling member as defined in claim 23.

25. An ostomy bag having a valve as defined in any of claims 1 to 22.
